# EUROPEAN PATENT APPLICATION

(11) **EP 4 847 046 A1**
(43) Date of publication of application: **02.12.2026**
(21) Application number: 25744606.2
(22) Date of filing: 22.01.2025
(51) Int. Cl.: A61K 47/68, A61K 45/06, C07K 16/28, C12N 15/13, A61K 39/395, A61K 31/704, A61P 35/00

(54) **USE OF ANTIBODY-DRUG CONJUGATE TARGETING NECTIN-4**

(30) Priority: 22.01.2024 CN 202410087558
(71) Applicant: CSPC Megalith Biopharmaceutical Co., Ltd., Shijiazhuang, Hebei 050035 (CN)
(72) Inventor: ZHANG, Liqiong, Shijiazhuang, Hebei, 050035 (CN); WANG, Juan, Shijiazhuang, Hebei, 050035 (CN); WAN, Xuechao, Shijiazhuang, Hebei, 050035 (CN); GE, Lan, Shijiazhuang, Hebei, 050035 (CN); SUN, Guilan, Shijiazhuang, Hebei, 050035 (CN); ZHANG, Jihao, Shijiazhuang, Hebei, 050035 (CN); HE, Wentian, Shijiazhuang, Hebei, 050035 (CN); ZHANG, Lixuan, Shijiazhuang, Hebei, 050035 (CN); LIU, Shengnan, Shijiazhuang, Hebei, 050035 (CN)
(74) Representative: Mewburn Ellis LLP
(86) International application number: PCT/CN2025/073891
(87) International publication number: WO 2025/157167

(57) **Abstract**

Provided is the use of an antibody-drug conjugate targeting Nectin-4 in the preparation of a drug for treating tumors; also provided are a tumor treatment therapeutic regimen and a safe effective dose of the drug.

## Description

### Cross-Reference to Related Applications

The present application claims the right of priority for Chinese Patent Application No. 202410087558.1 filed on January 22, 2024, the entire content of which is incorporated herein by reference in its entirety for all purposes.

### Technical Field

The present application relates to the field of biopharmaceuticals, specifically the use and therapeutic regimen of an antibody-drug conjugate targeting Nectin-4.

### Background Art

Nectins are novel cell adhesion molecules (CAMs) of the immunoglobulin superfamily (Ig SF), and are involved in intercellular interactions independently of Ca²⁺ and form intercellular adherens junctions (AJs). The Nectin protein family consists of four members: Nectin-1, Nectin-2, Nectin-3, and Nectin-4. Nectins 1-3 are widely expressed in normal human tissues. Nectins 1-2 are commonly found in immune organs (bone marrow, thymus, spleen, and lymph nodes), while Nectin-3 is mainly expressed in the testes and placenta. Unlike other members of the Nectin family, which are widely expressed in normal human tissues, Nectin-4 is highly expressed in normal embryonic and fetal tissues with minimal expression in healthy adult tissues. Abnormally high expression of Nectin-4 can be detected in a variety of cancer samples. Therefore, Nectin-4 is a reliable marker for a variety of cancers. In particular, it is expressed at high levels in urothelial carcinoma, cervical cancer, breast cancer, lung cancer, and pancreatic cancer.

As a member of the Nectin protein family, Nectin-4 is a type I transmembrane protein belonging to the immunoglobulin-like intercellular adhesion molecule. It participates in the formation of intercellular adherens junctions, mediates intercellular junctions, and is involved in regulating physiological processes including cell proliferation, differentiation, and migration. Although the exact molecular mechanisms of Nectin-4 in tumorigenesis and progression remain unclear, multiple studies have shown that Nectin-4 can induce angiogenesis, promote cancer cell proliferation and migration, induce epithelial-mesenchymal transition (EMT), and is associated with the progression of various cancers.

Based on the expression pattern and level of Nectin-4, it has become an "attractive" target for research on the treatment of various tumor diseases. To date, drug development targeting the Nectin-4 target mainly includes antibody-drug conjugates (ADCs), bicycle toxin conjugates (BTCs), bicycle peptides, monoclonal antibodies, and chimeric antigen receptor T-cell immunotherapy (CAR-T). Currently, the most advanced drug targeting Nectin-4 is Enfortumab vedotin (trade name: PADCEV^{®}), jointly developed by Astellas and Seagen. It is an ADC medicament obtained by conjugating enfortumab, a Nectin-4-targeted monoclonal antibody, with the microtubule-disrupting agent MMAE via a cleavable dipeptide linker. This medicament was approved by the FDA in 2019 primarily for the treatment of patients with locally advanced or metastatic urothelial carcinoma.

### Summary of the Invention

In a first aspect, the present application provides the use of an antibody-drug conjugate targeting Nectin-4 or a pharmaceutically acceptable salt thereof in the manufacture of a medicament for the treatment of a tumor (including a cancer), wherein the tumor is preferably a Nectin-4-positive tumor, and the antibody-drug conjugate is prepared by conjugating a drug-linker represented by formula I with an antibody targeting Nectin-4 or an antigen-binding fragment thereof:

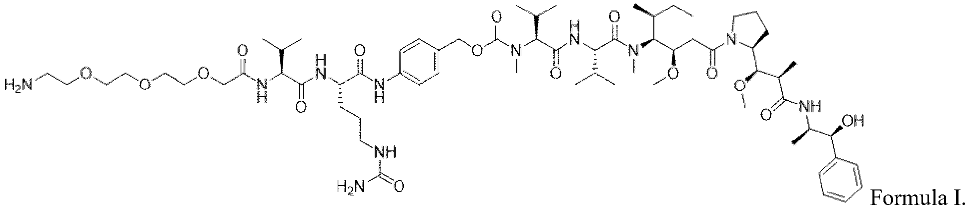

In a second aspect, the present application provides a method for treating a tumor, which method comprises administering to a subject or patient in need thereof a therapeutically effective amount of an antibody-drug conjugate or a pharmaceutically acceptable salt thereof, wherein the tumor is preferably a Nectin-4-positive tumor, and the antibody-drug conjugate is prepared by conjugating a drug-linker represented by formula I with an antibody targeting Nectin-4 or an antigen-binding fragment thereof:

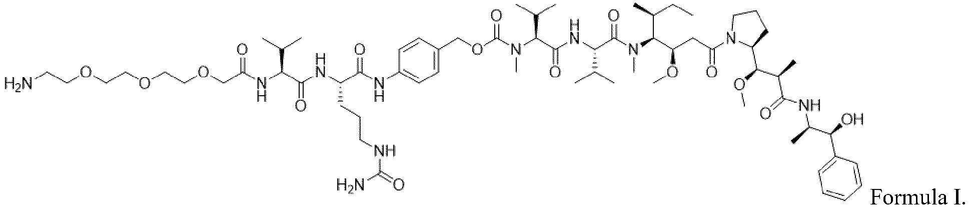

In a third aspect, the present application provides a pharmaceutical composition comprising (i) an antibody-drug conjugate targeting Nectin-4 or a pharmaceutically acceptable salt thereof and (ii) a pharmaceutically acceptable carrier, for use in the treatment of a tumor (including a cancer), wherein the tumor is preferably a Nectin-4-positive tumor, and the antibody-drug conjugate is prepared by conjugating a drug-linker represented by formula I with an antibody targeting Nectin-4 or an antigen-binding fragment thereof:

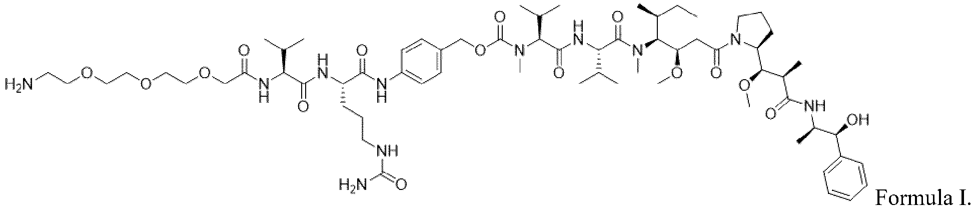

### Brief Description of the Drawings

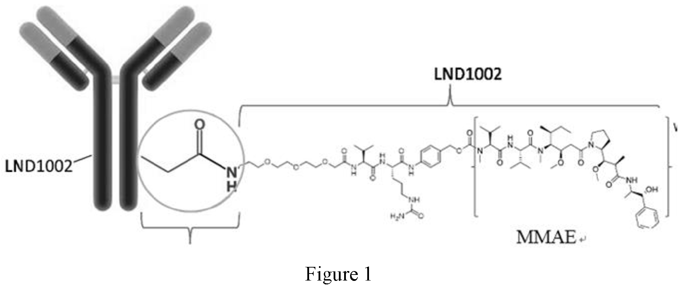

FIG. 1 shows a schematic structural diagram of the antibody-drug conjugate of the present application, in which LND1002 represents the moiety of the linker + drug molecule, and the circled part highlights the amide bond linking the linker and the antibody within the L&D moiety. The Nectin-4-targeted ADC shown in this figure is a site-specific antibody-drug conjugate. Each conjugate molecule is prepared by conjugating one molecule of MMAE with one anti-Nectin-4 monoclonal antibody molecule at the amino acid position numbered Q295 (per Kabat numbering) on each heavy chain via the linker (NH₂-(CH₂-CH₂-O)₃-CH₂-C(=O)-Val-Cit-pABC). The antibody is linked to the linker via a stable amide bond (isopeptide bond), and the resultant conjugate features an average drug-to-antibody ratio (DAR) of 2.0.
FIG. 2 shows the percentage change in target lesions based on the best overall response assessment in Example 1 of the present application.
FIG. 3 shows the Bayesian optimal interval (BOIN) design decision diagram used in the examples of the present application.
FIG. 4 shows the design dose escalation decision diagram used in the examples of the present application.

### Detailed Description of Embodiments

The specific embodiments and examples listed below are for the purpose of better illustrating the content of the present application, but the content of the present application is not limited to the specific embodiments and examples described. Non-essential improvements and adjustments made to the embodiments by those skilled in the art according to the above Summary of the Invention still fall within the scope of protection of the present application.

### Definition of Terms

Unless otherwise defined, all scientific and technical terms used herein have the same meaning as understood by those of ordinary skill in the art. Abbreviations for amino acid residues are the standard 3-letter and/or 1-letter codes used in the art to refer to one of the 20 commonly used L-amino acids.

Although the numerical ranges and parameters as shown in the broad scope of the present application are approximations, the numerical values as shown in specific embodiments are recited as precisely as possible. However, any numerical value inherently contains errors to some extent due to the standard deviation present in its respective measurements. Furthermore, all ranges disclosed herein should be understood to encompass any and all subranges contained therein. For example, the range "1 to 10" disclosed herein should be considered to include any and all subranges between the minimum value of 1 and the maximum value of 10 (inclusive); that is, all subranges that start with the minimum value of 1 or greater, such as 1 to 6.1, and subranges that end with the maximum value of 10 or less, such as 5.5 to 10 are included. Furthermore, any reference document referred to as "incorporated herein" should be understood to be incorporated in its entirety.

As used herein, the term "pharmaceutical composition" refers to a combination of at least one drug and optionally a pharmaceutically acceptable carrier or excipient, which are combined together to achieve a particular purpose. The types of pharmaceutically acceptable carriers are not particularly limited. Suitable buffers, stabilizers, surfactants, etc., can be added according to the ADC medicament formulation technology in this field. The type of pharmaceutically acceptable carriers used herein depends particularly on whether the composition according to the present application is formulated for oral, nasal, intradermal, subcutaneous, intramuscular or intravenous administration.

Broadly speaking, the term "antibody" can refer to an immunoglobulin molecule that can specifically bind to a target via at least one antigen recognition site located in the variable region of the immunoglobulin molecule. Therefore, the term encompasses intact antibodies/full-length antibodies, single-chain antibodies, or any antigen-binding fragment of an antibody (also known as an "antigen-binding portion"). When the terms "antibody" and "antigen-binding fragment" appear in the same context, the term "antibody" can be understood as the intact antibody relative to the term "antigen-binding fragment", and the two together correspond to the broad concept of antibody.

The term "full-length/intact antibody" refers to a protein that contains at least two heavy (H) chains and two light (L) chains interlinked by disulfide bonds. Each heavy chain comprises a heavy chain variable region (abbreviated as VH) and a heavy chain constant region comprising three domains CH1, CH2 and CH3. Each light chain comprises a light chain variable region (abbreviated as VL) and a light chain constant region comprising one domain CL. The VH and VL regions can be further subdivided into several regions of hypervariability termed complementarity determining regions (CDRs), interspersed with several more conserved regions termed framework regions (FRs). Each VH and VL can be composed of three CDRs and four FRs, which can be arranged from an amino terminus to a carboxyl terminus in the following order: FR1, CDR1, FR2, CDR2, FR3, CDR3, FR4. These variable regions of the heavy and light chains contain binding domains that interact with antigens. The constant region of an antibody can mediate the binding of the immunoglobulin to host tissues or factors, including various cells of the immune system (e.g., effector cells) and the first component (C1q) of the classical complement system. Chimeric or humanized antibodies are also encompassed in the antibodies according to the present application. The full-length/intact antibody can be an antibody of any class, such as IgD, IgE, IgG, IgA, or IgM (or subclasses of the above), but is not limited to any specific class. Immunoglobulins can be assigned to different classes based on the amino acid sequences of the heavy chain constant domains of the antibodies. Generally, there are five main classes of immunoglobulins: IgA, IgD, IgE, IgG, and IgM. Several of these classes can be further subdivided into subclasses (isotypes), such as IgG1, IgG2, IgG3, IgG4, IgA1, and IgA2. The heavy chain constant domains corresponding to different immunoglobulin classes are termed α, δ, ε, γ, and µ, respectively. The subunit structures and three-dimensional structures of different classes of immunoglobulins are well known.

The term "antigen-binding fragment" used herein refers to an antibody fragment, such as Fv, Fab, F(ab')₂, or Fab', or any fragment capable of having an increased half-life via chemical modification or incorporation into liposomes. The chemical modification is, for example, conjugation with poly(alkylene) glycol such as polyethylene glycol ("pegylation, PEGylated") (resulting in a pegylated fragment referred to as Fv-PEG, scFv-PEG, Fab-PEG, F(ab')₂-PEG or Fab'-PEG) ("PEG" stands for polyethylene glycol), and the fragment has Nectin-4 binding activity. Preferably, the antigen-binding fragment consists of or comprises partial sequences of the heavy chain variable region or light chain variable region of the parent antibody. The partial sequences are sufficient to retain the same binding specificity and adequate affinity as the parent antibody. Such functional fragment contains a minimum of 5 amino acids, preferably 10, 15, 25, 50 or 100 consecutive amino acids of the parent antibody sequence. Examples of the antigen-binding fragments include, but are not limited to: (1) Fab fragment, which can be a monovalent fragment having a VL-CL chain and a VH-CH1 chain; (2) F(ab')₂ fragment, which can be a divalent fragment having two Fab' fragments, wherein the two Fab' fragments are linked via a disulfide bridge in the hinge region (i.e., a dimer of Fab'); (3) Fv fragment having the VL and VH domains of a single arm of an antibody.

The complementarity determining regions (CDRs, usually CDR1, CDR2 and CDR3) are the regions within the variable region that exert the greatest impact on the affinity and specificity of an antibody. There are multiple common definitions for the CDR sequences of VH or VL, including the IMGT, Chothia and Kabat definitions. With regard to the variable region sequences of a given antibody, the CDR sequences within the VH and VL sequences can be identified according to the IMGT, Chothia or Kabat definitions. The CDRs in the present application are defined according to IMGT.

The term "humanized antibody" refers to an antibody obtained by grafting CDR sequences derived from another mammalian species, such as mouse germline, onto human framework sequences. To retain binding affinity, some residues in the framework (referred to as FR) regions may be modified. The humanized antibodies or fragments thereof according to the present application can be prepared by techniques known to those skilled in the art.

In the present application, the term "unit preparation" refers to the smallest subpackage of each pharmaceutical preparation comprising an appropriate amount of an active compound. For example, a unit preparation in capsules refers to one capsule, a unit preparation in tablets refers to one tablet, and a unit preparation in injections refers to one injection; this applies to other types of preparations.

In the present application, the term "therapeutically effective amount" refers to an amount that produces a therapeutic effect on a subject. For example, in a subject to which the amount is administered, the symptoms or states of a disease are mitigated, palliated, or eliminated, or the progression of the symptoms or states of a disease is delayed or inhibited, compared to a subject to which the amount is not administered.

In the present application, the term "best overall response" is defined as the best overall response at all time points per the Response Evaluation Criteria in Solid Tumors (RECIST) v1.1. When confirmation of complete response (CR)/partial response (PR) is not required: for a patient achieving efficacy assessed as stable disease (SD) in the first cycle, partial response (PR) in the second cycle, and progressive disease (PD) in the final cycle, the patient's best overall response shall be assessed as PR. When the best overall response is assessed as SD, the patient must meet the minimum duration from baseline as specified in the protocol. If the minimum duration criterion is not met, even if the best overall response is assessed as SD, it shall not be recognized, and the patient's best overall response shall be determined in subsequent assessments. For example, for a patient assessed as having SD in the first cycle and PD in the second cycle, but failing to meet the minimum duration of SD, the patient's best overall response shall be assessed as PD. Similarly, a patient who is lost to follow-up after being assessed as having SD in the first cycle shall be deemed non-evaluable. When confirmation of complete response (CR)/partial response (PR) is required: complete response or partial response can only be confirmed when the patient meets the criteria for partial response or complete response, and the response is reconfirmed at a subsequent time point (generally four weeks later).

As used herein, the term "standard of care", "best practice" and "standard therapy" refers to the treatment that is accepted by medical practitioners to be an appropriate, proper, effective, and/or widely used treatment for a given disease. The standard of care of a given disease depends on many different factors, including the biological effect of treatment, region or location within the body, patient status (e.g. age, weight, gender, hereditary risks, other disabilities, secondary conditions), toxicity, metabolism, bioaccumulation, therapeutic index, dosage, and other factors known in the art. Determining a standard of care for a disease is also dependent on establishing safety and efficacy in clinical trials as standardized by regulatory bodies such as the US Food and Drug Administration, International Council for Harmonisation, Health Canada, European Medicines Agency, Therapeutic Goods Administration, Central Drugs Standard Control Organization, National Medical Products Administration, Pharmaceuticals and Medical Devices Agency, Ministry of Food and Drug Safety, and the World Health Organization. The standard of care for a disease may include but is not limited to surgery, radiation, chemotherapy, targeted therapy, or immunotherapy.

In the present application, the term "Nectin-4-positive tumor" shall be construed in accordance with its ordinary meaning in the art, and generally refers to a tumor where Nectin-4 is expressed in at least a portion of tumor cells. The degree of Nectin-4 positivity of tumors can be analyzed qualitatively, quantitatively or semi-quantitatively by various methods known in the art. One exemplary method is to perform immunohistochemical analysis on solid tumor samples (e.g., biopsy specimens or post-operative sections), for example, with semi-quantitative presentation using the H-score. The H-score representing Nectin-4 expression in tumor cells can be expressed as the weighted sum of the percentages of stained cells: H-score = (3 × Percentage of strongly stained cells) + (2 × Percentage of moderately stained cells) + (1 × Percentage of weakly stained cells).

In general, the present application provides the use of an antibody-drug conjugate targeting Nectin-4 or a pharmaceutically acceptable salt thereof in the manufacture of a medicament for the treatment of a tumor (including a cancer), a method for treating a tumor (including a cancer) using an antibody-drug conjugate targeting Nectin-4 or a pharmaceutically acceptable salt thereof, and a pharmaceutical composition comprising an antibody-drug conjugate targeting Nectin-4 or a pharmaceutically acceptable salt thereof for use in the treatment of a tumor (including a cancer). In some non-limiting examples, the antibody-drug conjugate targeting Nectin-4 or the pharmaceutically acceptable salt thereof according to the present application exhibits lower free plasma MMAE and fewer treatment-related adverse events compared to PADCEV treatment.

In a first aspect, the present application provides the use of an antibody-drug conjugate targeting Nectin-4 or a pharmaceutically acceptable salt thereof in the manufacture of a medicament for the treatment of a tumor (including a cancer), wherein the tumor is preferably a Nectin-4-positive tumor, and the antibody-drug conjugate is prepared by conjugating a drug-linker represented by formula I with an antibody targeting Nectin-4 or an antigen-binding fragment thereof:

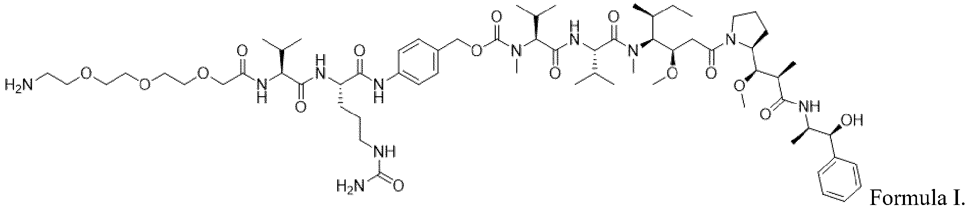

In some embodiments, the antibody or the antigen-binding fragment thereof comprises a heavy chain and a light chain, wherein
(i) the heavy chain comprises three CDR regions which are a CDR1-H region, a CDR2-H region and a CDR3-H region, and the CDR1-H region, CDR2-H region and CDR3-H region have the amino acid sequences as set forth in SEQ ID NOs: 1, 2 and 3, respectively; and/or
(ii) the light chain comprises three CDR regions which are a CDR1-L region, a CDR2-L region and a CDR3-L region, and the CDR1-L region, CDR2-L region and CDR3-L region have the amino acid sequences as set forth in SEQ ID NOs: 4, 5 and 6, respectively.

In some embodiments, the antibody is a human or murine antibody. In some embodiments, the antibody is a humanized antibody.

In some embodiments, the antibody or the antigen-binding fragment thereof comprises a heavy chain variable region and a light chain variable region, wherein the heavy chain variable region has an amino acid sequence as set forth in SEQ ID NO: 7, and the light chain variable region has an amino acid sequence as set forth in any one of SEQ ID NOs: 8-10.

In some embodiments, the antibody or the antigen-binding fragment thereof comprises a heavy chain constant region and a light chain constant region, wherein the heavy chain constant region has an amino acid sequence as set forth in SEQ ID NO: 11 or SEQ ID NO: 13, and the light chain constant region has an amino acid sequence as set forth in SEQ ID NO: 12.

In some embodiments, the antibody-drug conjugate has a structure as shown in FIG. 1. In some embodiments, the drug-linker represented by formula I is conjugated with an antibody targeting Nectin-4 or an antigen-binding fragment thereof via an amide bond, for example at the amino acid position numbered Q295 (per Kabat numbering) of the antibody heavy chain. In some embodiments, the antibody-drug conjugate has an average drug-to-antibody ratio (DAR) of 1-8 (not limited to an integer, but may also be a decimal), preferably an integer from 1 to 8, preferably 2, 4, 6, or 8, and more preferably 2.

In some embodiments, the tumor (including a cancer) is selected from the group consisting of cervical cancer (such as cervical adenocarcinoma or cervical squamous carcinoma), urothelial carcinoma (such as bladder cancer, renal pelvic carcinoma, carcinoma of urethra, or carcinoma of ureter), and breast cancer.

In some embodiments, the tumor is an advanced tumor.

In some embodiments, the urothelial carcinoma is locally advanced or metastatic urothelial carcinoma.

In some embodiments, the tumor is a tumor that has failed standard of care or prior treatment.

In some embodiments, the tumor is a tumor that is intolerant to Nectin-4 targeted therapy.

In some embodiments, the tumor is a tumor that has not previously received Nectin-4 targeted therapy.

In some embodiments, the antibody-drug conjugate or the pharmaceutically acceptable salt thereof is administered at a dosage ranging from about 0.001 mg/kg to about 1000 mg/kg per administration. In a preferred embodiment, the antibody-drug conjugate or the pharmaceutically acceptable salt thereof is administered at a dosage ranging from about 0.01 mg/kg to about 100 mg/kg per administration. In a preferred embodiment, the antibody-drug conjugate or the pharmaceutically acceptable salt thereof is administered at a dosage ranging from about 0.02 mg/kg to about 50 mg/kg per administration.

In some embodiments, the antibody-drug conjugate or the pharmaceutically acceptable salt thereof is administered in a single dose or as divided doses.

In some embodiments, the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg, or a range formed by any two of the above values. In some embodiments, the dosage per administration ranges from about 1.8 mg/kg to about 4.5 mg/kg, about 1.8 mg/kg to about 2.5 mg/kg, about 2.5 mg/kg to about 3.0 mg/kg, about 3.0 mg/kg to about 3.5 mg/kg, about 3.5 mg/kg to about 4.0 mg/kg, or about 4.0 mg/kg to about 4.5 mg/kg.

In some embodiments, the antibody-drug conjugate or the pharmaceutically acceptable salt thereof is administered at a frequency of once weekly (QW), once every two weeks (Q2W), once every three weeks (Q3W), or once every four weeks (Q4W).

In some embodiments, the dosing regimen of the antibody-drug conjugate or the pharmaceutically acceptable salt thereof is once every three weeks (Q3W), with a treatment cycle of 21 days.

In some embodiments, the dosing regimen of the antibody-drug conjugate or the pharmaceutically acceptable salt thereof is once every four weeks (Q4W), with a treatment cycle of 28 days.

In some embodiments, the dosing regimen of the antibody-drug conjugate or the pharmaceutically acceptable salt thereof is once every two weeks (Q2W), with a treatment cycle of 14 or 28 days.

In some embodiments, the dosing regimen of the antibody-drug conjugate or the pharmaceutically acceptable salt thereof is once weekly (QW) for three consecutive weeks, followed by a one-week break, with a treatment cycle of 28 days.

In some embodiments, the dosing regimen of the antibody-drug conjugate or the pharmaceutically acceptable salt thereof is once weekly (QW) for two consecutive weeks, followed by a one-week break, with a treatment cycle of 21 days.

In some embodiments, the antibody-drug conjugate or the pharmaceutically acceptable salt thereof is administered for 1, 2, 3 or more treatment cycles.

In some embodiments, the medicament contains a therapeutically effective amount of the antibody-drug conjugate or the pharmaceutically acceptable salt thereof. In some embodiments, the therapeutically effective amount is about 0.001 to about 1000 mg.

The administration route of the antibody-drug conjugate or the pharmaceutically acceptable salt thereof is not particularly limited. Representative administration routes can be oral administration, administration by injection, local administration, or *ex vivo* administration. Accordingly, the medicament is prepared into a clinically acceptable preparation using conventional excipients and processes, such as an oral preparation, an injectable preparation, a topical preparation, or an external preparation. In some embodiments, the injectable preparations are preferably powder for injection. These preparations can be prepared using preparation techniques known to those of ordinary skill in the art.

In some embodiments, based on the antibody-drug conjugate, a unit preparation of the medicament contains the antibody-drug conjugate or the pharmaceutically acceptable salt thereof in an amount of about 0.001 mg to about 1000 mg, preferably about 1 mg to about 500 mg, or about 5 mg to about 400 mg, or about 10 mg to about 300 mg, or about 10 mg to about 250 mg, or about 10 mg to about 200 mg, or about 20 mg to about 200 mg, more preferably about 10 mg to about 160 mg, or about 20 mg to about 160 mg, or about 80 mg to about 160 mg; or about 120 mg to about 240 mg, or about 160 mg to about 220 mg; for example, about 10 mg, about 20 mg, about 30 mg, about 40 mg, about 60 mg, about 80 mg, about 100 mg, about 120 mg, about 140 mg, about 160 mg, more preferably about 10 mg, about 20 mg, about 40 mg, about 80 mg, about 120 mg, or about 160 mg, further more preferably about 20 mg, about 40 mg, about 80 mg, about 120 mg, about 160 mg, or about 240 mg, or further more preferably about 10 mg or about 40 mg.

In some embodiments, based on the antibody-drug conjugate, a unit preparation of the medicament contains the antibody-drug conjugate or the pharmaceutically acceptable salt thereof in an amount of about 0.001 mg to about 1000 mg, preferably about 90 mg to 350 mg, for example: about 90 mg, about 100 mg, about 110 mg, about 120 mg, about 130 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, about 300 mg, about 305 mg, about 310 mg, about 320 mg, about 333 mg, about 340 mg, or about 350 mg.

In a second aspect, the present application provides a method for treating a tumor (including a cancer), which method comprises administering to a subject or patient a therapeutically effective amount of an antibody-drug conjugate targeting Nectin-4 or a pharmaceutically acceptable salt thereof, wherein the tumor is preferably a Nectin-4-positive tumor, and the antibody-drug conjugate is prepared by conjugating a drug-linker represented by formula I with an antibody targeting Nectin-4 or an antigen-binding fragment thereof:

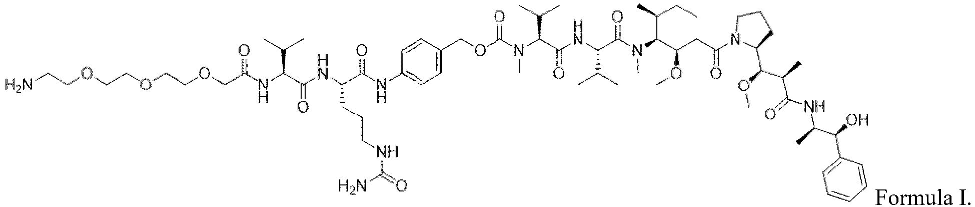

The embodiments and technical features described in the first aspect regarding antibodies or antigen-binding fragments thereof, antibody-drug conjugates, tumors, pharmaceutical dosage forms, drug dosages and dosing regimens shall likewise apply to the second aspect provided that there is no conflict. In some embodiments, the subject or patient is a subject or patient that has failed standard of care or prior treatment.

In some embodiments, the subject or patient is a subject or patient that is intolerant to Nectin-4 targeted therapy.

In some embodiments, the subject or patient is a subject or patient that has not previously received Nectin-4 targeted therapy.

In some embodiments, the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg.

In some embodiments, the antibody-drug conjugate or the pharmaceutically acceptable salt thereof is administered at a frequency of QW, Q2W, Q3W, or Q4W.

In some embodiments, the antibody-drug conjugate or the pharmaceutically acceptable salt thereof is administered in a single dose or as divided doses.

In some embodiments, the antibody-drug conjugate or the pharmaceutically acceptable salt thereof is administered at a frequency of QW, and the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg, or a range formed by any two of the above values. In some embodiments, the dosage per administration ranges from about 1.8 mg/kg to about 4.5 mg/kg, about 1.8 mg/kg to about 2.5 mg/kg, about 2.5 mg/kg to about 3.0 mg/kg, about 3.0 mg/kg to about 3.5 mg/kg, about 3.5 mg/kg to about 4.0 mg/kg, or about 4.0 mg/kg to about 4.5 mg/kg.

In some embodiments, the antibody-drug conjugate or the pharmaceutically acceptable salt thereof is administered at a frequency of Q2W, and the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg, or a range formed by any two of the above values. In some embodiments, the dosage per administration ranges from about 1.8 mg/kg to about 4.5 mg/kg, about 1.8 mg/kg to about 2.5 mg/kg, about 2.5 mg/kg to about 3.0 mg/kg, about 3.0 mg/kg to about 3.5 mg/kg, about 3.5 mg/kg to about 4.0 mg/kg, or about 4.0 mg/kg to about 4.5 mg/kg.

In some embodiments, the antibody-drug conjugate or the pharmaceutically acceptable salt thereof is administered at a frequency of Q3W, and the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg, or a range formed by any two of the above values. In some embodiments, the dosage per administration ranges from about 1.8 mg/kg to about 4.5 mg/kg, about 1.8 mg/kg to about 2.5 mg/kg, about 2.5 mg/kg to about 3.0 mg/kg, about 3.0 mg/kg to about 3.5 mg/kg, about 3.5 mg/kg to about 4.0 mg/kg, or about 4.0 mg/kg to about 4.5 mg/kg.

In some embodiments, the antibody-drug conjugate or the pharmaceutically acceptable salt thereof is administered at a frequency of Q4W, and the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg, or a range formed by any two of the above values. In some embodiments, the dosage per administration ranges from about 1.8 mg/kg to about 4.5 mg/kg, about 1.8 mg/kg to about 2.5 mg/kg, about 2.5 mg/kg to about 3.0 mg/kg, about 3.0 mg/kg to about 3.5 mg/kg, about 3.5 mg/kg to about 4.0 mg/kg, or about 4.0 mg/kg to about 4.5 mg/kg.

In some embodiments, the antibody-drug conjugate or the pharmaceutically acceptable salt thereof is administered at a frequency of Q2W, and the antibody-drug conjugate or the pharmaceutically acceptable salt thereof is administered at a dosage ranging from about 2.0 mg/kg to 3.0 mg/kg per administration, based on the weight of the antibody-drug conjugate, more preferably about 2.4 mg/kg or about 2.7 mg/kg.

In some embodiments, the antibody-drug conjugate or the pharmaceutically acceptable salt thereof is administered at a frequency of Q3W, and the antibody-drug conjugate or the pharmaceutically acceptable salt thereof is administered at a dosage ranging from about 2.0 mg/kg to 4.0 mg/kg per administration, based on the weight of the antibody-drug conjugate, more preferably about 2.4 mg/kg, 2.7 mg/kg, about 3.6 mg/kg, or 4.5 mg/kg.

In some embodiments, based on the weight of the antibody-drug conjugate, the therapeutically effective amount of the antibody-drug conjugate or the pharmaceutically acceptable salt thereof is about 1 mg to about 500 mg, preferably about 5 mg to about 400 mg, or about 10 mg to about 300 mg, or about 10 mg to about 250 mg, or about 10 mg to about 200 mg, or about 20 mg to about 200 mg, more preferably about 10 mg to about 160 mg, or about 20mg to about 160 mg, or about 80 mg to about 160 mg; or about 120 mg to about 240 mg, or about 160 mg to about 220 mg; for example, about 10 mg, about 20 mg, about 40 mg, about 60 mg, about 80 mg, about 100 mg, about 120 mg, about 140 mg, or about 160 mg, more preferably about 10 mg, about 20 mg, about 40 mg, about 80 mg, about 120 mg, or about 160 mg, further more preferably about 20 mg, about 40 mg, about 80 mg, about 120 mg, about 160 mg, or about 240 mg, or further more preferably about 10 mg or about 40 mg.

In some embodiments, the therapeutically effective amount is about 90 mg to 350 mg, for example: about 90 mg, about 100 mg, about 110 mg, about 120 mg, about 130 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, about 300 mg, about 305 mg, about 310 mg, about 320 mg, about 333 mg, about 340 mg, or about 350 mg.

In some embodiments, the antibody-drug conjugate or the pharmaceutically acceptable salt thereof is administered by intravenous injection. In some embodiments, the antibody-drug conjugate or the pharmaceutically acceptable salt thereof is infused intravenously over 60±5 minutes.

In a third aspect, the present application provides a pharmaceutical composition comprising (i) an antibody-drug conjugate targeting Nectin-4 or a pharmaceutically acceptable salt thereof and (ii) a pharmaceutically acceptable carrier, for use in the treatment of a tumor (including a cancer), wherein the tumor is preferably a Nectin-4-positive tumor, and the antibody-drug conjugate is prepared by conjugating a drug-linker represented by formula I with an antibody targeting Nectin-4 or an antigen-binding fragment thereof:

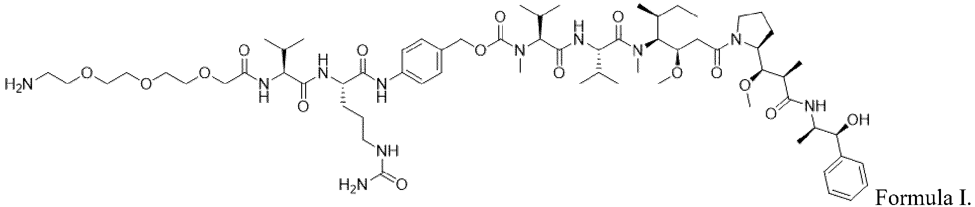

The embodiments and technical features described in the first and second aspects regarding antibodies or antigen-binding fragments thereof, antibody-drug conjugates, tumors, patients, pharmaceutical dosage forms, drug dosages and dosing regimens shall likewise apply to the third aspect provided that there is no conflict.

In some embodiments, the pharmaceutical composition comprises: (i) the antibody-drug conjugate, (ii) a buffer, (iii) a stabilizer, and (iv) a surfactant. In some embodiments, the surfactant is Polysorbate 20 or Polysorbate 80, preferably Polysorbate 20. In some embodiments, the stabilizer is sucrose or trehalose, preferably trehalose. In some embodiments, the buffer is histidine-histidine hydrochloride, succinic acid-sodium succinate, or citric acid-sodium citrate, preferably histidine-histidine hydrochloride. In some specific embodiments, the pharmaceutical composition comprises: histidine-histidine hydrochloride: 5-30 mM, Polysorbate 20 or Polysorbate 80: 0.02 wt%-0.08 wt%, trehalose: 4 wt%-8 wt%, and the antibody-drug conjugate: 5-50 mg/ml, pH 5.0-6.5. In some specific embodiments, the pharmaceutical composition comprises: histidine-histidine hydrochloride: 10-30 mM, Polysorbate 20 or Polysorbate 80: 0.02 wt%-0.04 wt%, trehalose: 5 wt%-7 wt%, and the antibody-drug conjugate: 10-20 mg/ml, pH 5.0-6.0. In some specific embodiments, the pharmaceutical composition comprises: histidine-histidine hydrochloride: 20 mM, Polysorbate 20: 0.02 wt%, trehalose: 5.5 wt%, and the antibody-drug conjugate: 10 mg/ml, pH 6.0. In some embodiments, the pharmaceutical composition further comprises water. In some embodiments, the pharmaceutical composition is a liquid preparation, a lyophilized preparation, or a powder for injection, preferably a liquid preparation or a lyophilized preparation. The embodiments and technical features described in this paragraph regarding the pharmaceutical composition also apply to the medicament described in the first aspect and the therapeutic agent in the method of the second aspect.

SWY2001-Ab3-LND1002 is a non-limiting example of the antibody-drug conjugate of the present application, in which the antibody moiety is humanized antibody 3 SWY2001-Ab3, and in the antibody, the heavy chain variable region is SWY2001-Ab3-VH (SEQ ID NO: 7), the light chain variable region is SWY2001-Ab3-VL (SEQ ID NO: 10), the heavy chain constant region is HC1 (SEQ ID NO: 11), and the light chain constant region is LC (SEQ ID NO: 12). For the structure of SWY2001-Ab3-LND1002, reference can be made to FIG. 1 of the present application and related description. The preparation methods of the antibody in SWY2001-Ab3-LND1002 and the antibody-drug conjugate itself follow the methods described in WO 2023/025243 A1, the entire content of which is incorporated herein by reference for all purposes. The preparation method of the test preparation of SWY2001-Ab3-LND1002 follows the method described in WO2024/179453 A1, the entire content of which is incorporated herein by reference for all purposes.

### Example 1: An open-label, single-arm, multicenter phase I clinical trial to evaluate the safety, tolerability, pharmacokinetic profiles and preliminary efficacy of SWY2001-Ab3-LND1002 in patients with advanced solid tumors

### 1. Drug information and experimental protocol

### 1.1 Drug information

Name of investigational drug: SWY2001-Ab3-LND1002 (recombinant humanized antihuman Nectin-4 monoclonal antibody-MMAE conjugate for injection).
Specification: 30 mg/vial.
Dosage form: powder for injection.
Storage conditions: store at 2°C-8°C, protected from light; keep away from light prior to use and avoid shaking.

### 1.2 Experimental objectives

### Primary objectives:

To evaluate the safety and tolerability of SWY2001-Ab3-LND1002 in patients with advanced solid tumors, and to determine the maximum tolerated dose (MTD) (if applicable) and the recommended phase II dose (RP2D) of SWY2001-Ab3-LND1002.

### Secondary objectives:

To evaluate the pharmacokinetic (PK) profiles of SWY2001-Ab3-LND1002 in patients with advanced solid tumors;
to evaluate the immunogenicity of SWY2001-Ab3-LND1002 in patients with advanced solid tumors;
to preliminarily evaluate the efficacy of SWY2001-Ab3-LND1002 in treating advanced solid tumors positive for Nectin-4 expression.

### 1.3 Overall study design

Sample size: a total of no more than 429 patients.
Dose-escalation phase: no more than 36 patients enrolled.
PK expansion phase: 2-3 dose groups were selected for PK expansion, with each dose group expanded to 8-12 subjects (including those in the same dose group of the dose-escalation phase who have completed all PK blood collections prior to Cycle 2 administration).
Cohort expansion phase: 55-97 subjects were enrolled in cohort A. For cohorts B-CE, 20 to 50 subjects were planned to be enrolled per cohort for exploratory study objectives.

Dose-escalation and PK expansion phases for urothelial carcinoma with severe renal insufficiency: 6 to 12 subjects were enrolled in the dose-escalation phase, and a maximum of 15 subjects (including those from the dose-escalation phase) were enrolled in the expansion phase.

### 1.4 Primary study endpoints

Primary endpoints: Safety endpoints: incidence and frequency of adverse events (AEs), serious adverse events (SAEs), and dose-limiting toxicity (DLT); MTD (if applicable), RP2D.
Secondary endpoints: PK endpoints: plasma drug levels and/or PK parameters of toxin-conjugated antibody, total antibody, free MMAE and MMAE derivatives after single and repeated dosing of SWY2001-Ab3-LND1002, including but not limited to AUC₀₋ₜ, AUC_{0-∞}, Cₘₐₓ, Tₘₐₓ, t_{1/2}, CL, Vss, MRT, C_{max,ss}, C_{min,ss}, T_{max,ss}, AUCₛₛ, R_{ac}, etc.;

Immunogenicity: incidence of anti-drug antibodies (ADAs) against SWY2001-Ab3-LND1002;
Efficacy endpoints: objective response rate (ORR), duration of response (DoR), disease control rate (DCR), progression-free survival (PFS) and overall survival (OS).

### 1.5 Experimental design

This study was an open-label, single-arm, multicenter phase I clinical trial, divided into four phases: dose escalation phase, PK expansion phase, cohort expansion phase, and dose escalation and PK expansion phases for urothelial carcinoma with severe renal insufficiency. 1.5.1 Dose-escalation phase

The starting dose of SWY2001-Ab3-LND1002 was 0.2 mg/kg and administered by intravenous infusion once every 3 weeks (Q3W), with a treatment cycle of 21 days.

Dose escalation was conducted in this study using the Bayesian Optimal Interval (BOIN) design with accelerated titration.

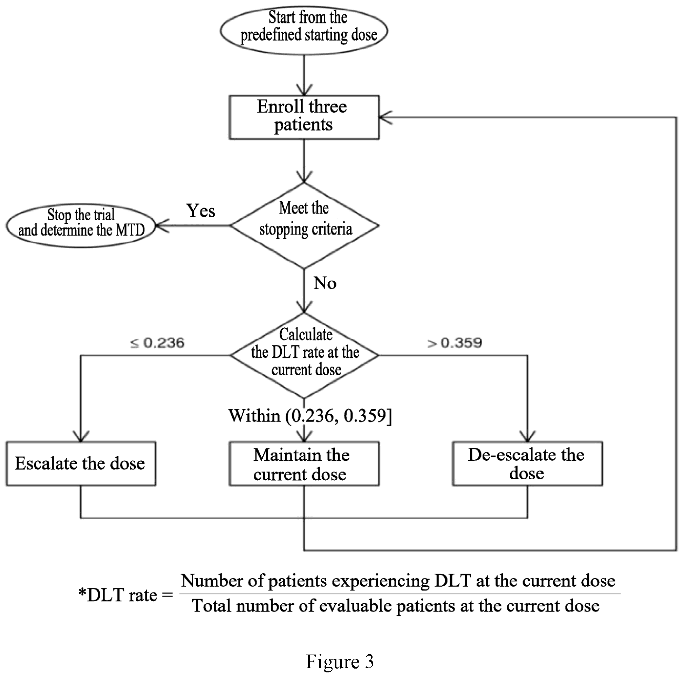

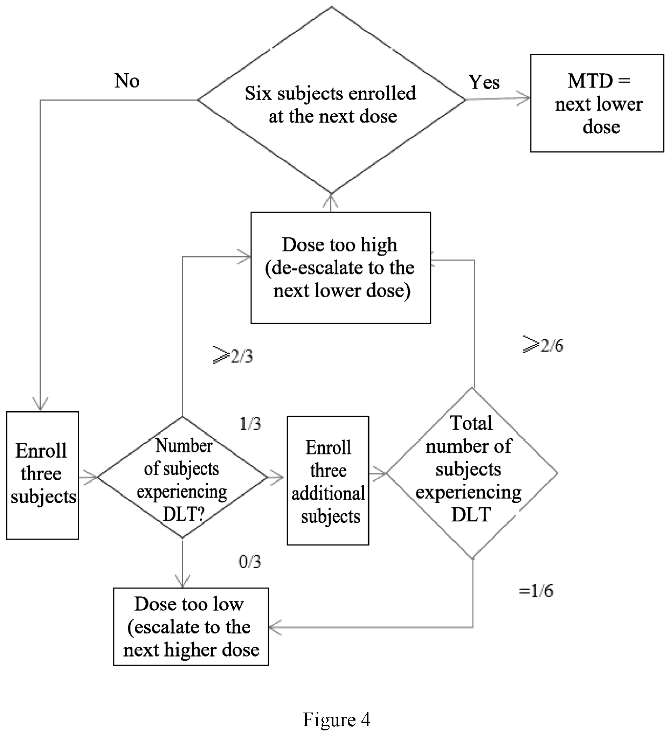

BOIN design phase: Six dose groups were predefined: 0.2 mg/kg, 0.6 mg/kg, 1.2 mg/kg, 1.8 mg/kg, 2.7 mg/kg, and 3.6 mg/kg. The 0.2 mg/kg group was the accelerated titration dose group. The target toxicity rate was 0.3, and the maximum sample size for the "BOIN" design was 36 subjects. Three subjects were enrolled per group to receive treatment. Cycle 1 administration (C1) was used as the observation window for DLT, and dose escalation/de-escalation decisions were made based on DLT incidence. As shown in FIG. 3 and FIG. 4, dose escalation/de-escalation decisions in the BOIN design followed the optimized rules below:
1. If the estimated dose-limiting toxicity (DLT) rate at the current dose was ≤ 0.236, the dose was escalated to the next higher dose level. The estimated DLT rate at the current dose was calculated as the ratio of the number of subjects experiencing DLT at the current dose level to the number of subjects who receive treatment at the current dose.
2. If the estimated DLT rate at the current dose was > 0.359, the dose was de-escalated to the next lower dose level.
3. Otherwise, the current dose was maintained.

The specific steps for conducting clinical trials using the BOIN design were as follows:
1. Accelerated titration was conducted in the accelerated titration dose group (0.2 mg/kg) according to the following steps: the first subject was assigned to this dose level. If the subject did not experience dose-limiting toxicity (DLT), the accelerated titration was completed, and subsequent subjects were sequentially enrolled in the 0.6 mg/kg dose group to receive treatment following steps 2 and 3, with 3 subjects per group. If the subject experienced DLT, two additional subjects were enrolled and treated at the current dose. Subsequently, subsequent subjects were sequentially enrolled following steps 2 and 3, with 3 subjects per group.
2. Doses for next group of subjects were assigned per the dose escalation/de-escalation rule shown in the table below. The rule is a tabular representation of the aforementioned rule. When the table below is used, please note the following:

**Table 1: Dose escalation/de-escalation decision table for Bayesian optimal interval (BOIN) design**

| | | | | | | | | | | | | |
|---|---|---|---|---|---|---|---|---|---|---|---|---|
| | | | | | | | | | | | | |

| | **1** | **2** | **3** | **4** | **5** | **6** | **7** | **8** | **9** | **10** | **11** | **12** |
|---|---|---|---|---|---|---|---|---|---|---|---|---|
| **Total number of evaluable subjects treated at the current dose** | 1 | 2 | 3 | 4 | 5 | 6 | 7 | 8 | 9 | 10 | 11 | 12 |
| **Escalate dose if number of subjects with DLT ≤** | 0 | 0 | 0 | 0 | 1 | 1 | 1 | 1 | 2 | 2 | 2 | 2 |
| **De-escalate dose if number of subjects with DLT ≥** | 1 | 1 | 2 | 2 | 2 | 3 | 3 | 3 | 4 | 4 | 4 | 5 |
| **Eliminate dose if number of subjects with DLT ≥** | NA | NA | 3 | 3 | 4 | 4 | 5 | 5 | 5 | 6 | 6 | 7 |
| Notes: If no decision (i.e., dose escalation, de-escalation, or elimination) is triggered, the next group of subjects will be treated at the current dose. | | | | | | | | | | | | |
| "NA" indicates that the dose should have been used to treat at least 3 evaluable subjects before being eliminated. | | | | | | | | | | | | |

a. "Elimination" refers to the elimination of the current dose and higher doses from the trial. The eliminated dose is an overtoxic dose and will no longer be used to treat any newly enrolled subjects.
b. If the current dose is eliminated, automatic dose de-escalation is conducted, and the subjects are treated at the next lower dose level. If the lowest dose is eliminated, the trial will be terminated early to ensure subject safety. In this case, the maximum tolerated dose (MTD) cannot be determined.
c. If no decision (i.e., dose escalation, de-escalation, or elimination) condition is met, the next group of subjects will continue to be treated at the current dose.
d. If the current dose is the lowest dose but a dose de-escalation is required according to the rule, new subjects will continue to be treated at the current lowest dose. If the number of subjects experiencing DLT reaches the elimination boundary, the trial will be terminated early to ensure subject safety.
e. If the current dose is the maximum dose but a dose escalation is still required according to the rule, newly enrolled subjects will continue to be treated at the current maximum dose, or the SMC will decide whether to explore a higher dose.

3. Step 2 was repeated until the predefined maximum sample size of 36 was reached, or the number of subjects treated at the current dose was ≥ 12 and the current decision was to maintain the current dose according to the escalation/de-escalation rule, the trial would be terminated early.

At the end of the trial, the maximum tolerated dose (MTD) was determined using isotonic regression based on all data from enrolled subjects. To better identify MTD, one or more dose groups may also be added between the maximum escalated dose group and the next lower dose group for DLT assessment. If necessary, the principal investigator and the sponsor shall jointly discuss and decide whether to add additional dose groups of SWY2001-Ab3-LND1002 (such as one or more dose groups between the maximum escalated dose group and the next lower dose group) and/or adjust the dosing frequency (such as once every two weeks (Q2W)) based on the obtained safety, tolerability, PK, immunogenicity data and efficacy information.

### 1.5.2. Dose-expansion phase

Based on the safety, tolerability, and PK data from different dose groups during the dose-escalation phase, 2-3 dose groups were selected for PK expansion. Each dose group was planned to expand to 8-12 subjects (including those in the same dose group of the dose-escalation phase who have completed all PK blood collections prior to Cycle 2 administration). Meanwhile, if the dose group in the dose-escalation phase completed the safety assessment and was deemed tolerable, enrollment of subjects for the PK expansion phase could commence concurrently with the dose group.

### 1.5.3 Cohort expansion phase

For the cohort expansion phase, subjects were divided into three cohorts according to tumor type, namely:
cohort A: urothelial carcinoma;
cohort B: cervical cancer;
cohort C: other tumors.

The recommended dose of SWY2001-Ab3-LND1002 determined in the dose-escalation and PK expansion phases was used in this phase. 55-97 subjects were planned to be enrolled in cohort A. For cohorts B-E, 20 to 50 subjects were planned to be enrolled per cohort for exploratory study objectives.

Based on preliminary research results and the sponsor's research and development strategy, the cohorts could be adjusted (e.g., adding or removing cohorts) during the study following discussions between the sponsor and investigator.

### 1.5.4 Dose-escalation and PK expansion phases for urothelial carcinoma with severe renal insufficiency

The subjects enrolled in this phase were urothelial carcinoma patients with severe renal insufficiency. Severe renal insufficiency was defined as creatinine clearance ≥ 15 mL/min and < 30 mL/min, calculated using the Cockcroft-Gault formula (i.e., creatinine clearance = {(140-age [years]) × body weight [kg]/(72 × serum creatinine [mg/dL])} × 0.85 [if female]) or based on 24-hour urine testing. Subjects first underwent dose escalation (using a 3+3 design), starting at the next lower dose relative to the recommended dose of SWY2001-Ab3-LND1002 determined during the preceding dose-escalation and PK expansion phases. Upon completion of safety assessment during the DLT observation period (i.e., Cycle 1 administration of the study), subject enrollment of the next cohort was determined in accordance with the dose escalation and de-escalation rule of the 3+3 design. The 3+3 dose escalation followed the principles below (see FIG. 3 for details):
The maximum escalated dose in this phase did not exceed the recommended dose of SWY2001-Ab3-LND1002 established during the preceding dose-escalation and PK expansion phases. Upon completion of the dose-escalation trial, the maximum tolerated dose was selected for the expansion phase (a maximum of 15 subjects were enrolled in the dose-escalation and expansion phases) to further evaluate the safety and PK of SWY2001-Ab3-LND1002 in urothelial carcinoma patients with severe renal insufficiency.

All subjects in this study underwent safety assessment as per the protocol after receiving their first study treatment, and PK and immunogenicity blood samples were collected after a single dosing and during repeated dosing. In addition, tumor imaging assessments were conducted every 6 weeks within the first 24 weeks following the initiation of study treatment per the Response Evaluation Criteria in Solid Tumors (RECIST) v1.1. After 24 weeks, tumor imaging assessments were conducted every 9 weeks, and an additional tumor imaging assessment was conducted upon treatment completion or study withdrawal (if no tumor imaging assessment was conducted within the preceding 4 weeks). All subjects received continuous treatment with SWY2001-Ab3-LND1002 (Q3W) until the treatment discontinuation criteria specified in the protocol were met, after which they entered the follow-up period.

Throughout the study, SMC continuously reviewed the safety of the investigational drug and made decisions on study-related issues such as study enrollment, dose escalation, dose group adjustment, expansion start time, and expansion dose recommendations.

### 2. Inclusion criteria and exclusion criteria

### 2.1 Inclusion criteria

Subjects must meet all of the following inclusion criteria to be enrolled in this study:
1. Age ≥ 18 years, either gender;
2. Patients with histologically confirmed advanced solid tumors who have failed to or are intolerant to standard of care, have no available standard of care, or decline standard of care;
3. Able to provide archived or fresh tumor tissue with positive Nectin-4 expression [based on immunohistochemistry (IHC) detection in a central laboratory], and for patients with urothelial carcinoma, Nectin-4 expression status is not restricted, but tumor tissue must still be provided;
4. Have at least one measurable lesion confirmed by CT or MRI per the Response Evaluation Criteria in Solid Tumors (RECIST) v1.1;
5. Eastern Cooperative Oncology Group (ECOG) performance status scores of 0-1;
6. Life expectancy ≥ 3 months;
7. Major organ function meets the criteria specified in Table 2 below within 7 days prior to treatment:

**Table 2.**

| **System** | **Laboratory parameters** |
|---|---|
| **Hematology:** No transfusion of blood components, or administration of human granulocyte colony-stimulating factor (G-CSF), thrombopoietin (TPO), interleukin-11, or erythropoietin (EPO) within 2 weeks prior to administration of the investigational drug | |
| Absolute neutrophil count (ANC) | ≥ 1.5×10⁹ /L |
| Platelet count (PLT) | ≥100×10⁹ /L |
| Hemoglobin (HGB) | ≥ 90 g/L or ≥ 5.6 mmol/L |

| **Renal function** | |
|---|---|
| Creatinine clearance (CrCL) | Subjects in dose-escalation, PK expansion and cohort expansion phases: ≥ 30 mL/min, calculated using the Cockcroft-Gault formula or based on 24-hour urine testing; |
| | subjects in dose-escalation and PK expansion phases for urothelial carcinoma with severe renal insufficiency: ≥ 15 mL/min and < 30 mL/min, calculated using the Cockcroft-Gault formula or based on 24-hour urine testing |

| **Liver function** | |
|---|---|
| Total bilirubin (TBIL) | ≤ 1.5×ULN; ≤ 3×ULN is permitted for patients with Gilbert's syndrome |
| Alanine aminotransferase (ALT) and aspartate aminotransferase (AST) | ≤ 2.5×ULN; ≤ 5×ULN for patients with liver metastasis; for subjects in dose-escalation and expansion phases for urothelial carcinoma with renal insufficiency, ALT and AST ≤ 1.5×ULN |

| **Coagulation function** | |
|---|---|
| Activated partial thromboplastin time (APTT) | ≤1.5×ULN |
| International normalized ratio (INR) | ≤1.5×ULN |

8. Women of childbearing potential (WOCBP) must have a negative serum pregnancy test within 7 days prior to the first dose of the investigational drug; patients and their partners must agree to use adequate contraception from the date of signing the informed consent form until 6 months after the last dose; and during this period, female subjects must not breastfeed, and male subjects must not donate sperm;
9. Subjects must voluntarily participate in this clinical study, understand the study procedures, and be able to sign the written informed consent form.

### 2.2 Exclusion criteria

1. Active central nervous system metastasis and/or leptomeningeal metastasis. Active central nervous system metastasis includes symptomatic (pre-existing or newly developed) and asymptomatic (pre-existing or newly developed) central nervous system metastasis requiring treatment. Subjects with central nervous system metastasis limited to the supratentorial region and/or cerebellum (i.e., no metastasis in the midbrain, pons, medulla oblongata or spinal cord) who have received local therapy, have stable neurological symptoms for at least 2 weeks prior to the first use of the investigational drug, and do not require steroid therapy or receive prednisone (or equivalent steroid) at a dose ≤ 10 mg/day may participate in the study;
2. Prior treatment with Nectin-4 ADC medicament containing MMAE toxin;
3. Adverse events from previous anti-tumor therapy have not resolved to ≤ Grade 1 per NCI-CTCAE v5.0 (alopecia excluded; based on the investigator's judgment and after consultation with the sponsor, some tolerable chronic Grade 2 toxicities may be excluded);
4. Any severe and/or uncontrolled concomitant disease that may prevent a patient from participating in the study:
   (1) History of severe cardiovascular diseases within 6 months prior to the first dose of the investigational drug, including but not limited to:
      1) Severe cardiac rhythm or conduction abnormalities, such as ventricular arrhythmia and third-degree atrioventricular block, requiring clinical intervention; Fridericia-corrected QT interval > 480 ms (Fridericia formula: QTcF = QT/RR^{0.33}, RR = 60/heart rate);
      2) History of myocardial infarction, unstable angina, angioplasty or coronary artery bypass surgery;
      3) Heart failure classified as Class III or above by the New York Heart Association (NYHA); left ventricular ejection fraction (LVEF) < 50% as detected during screening.
   (2) Other clinically significant diseases:
      1) HbA1C ≥ 8%;
      2) Active keratitis and corneal ulcer;
      3) ≥ Grade 2 Neuropathy prior to the first dose of the investigational drug;
      4) Severe infection, including but not limited to bacteremia requiring hospitalization and severe pneumonia, within 4 weeks prior to the first dose of the investigational drug; active infection ≥ grade 2 per CTCAE v5.0 requiring systemic antibiotic, antiviral or antifungal therapy within 2 weeks prior to the first dose of the investigational drug;
      5) Active HBV or HCV infection (HBV-DNA ≥ 2000 IU/mL, HCV antibody positive with HCV RNA above the lower limit of quantification);
      6) History of immunodeficiency (including positive HIV test results, and other acquired or congenital immunodeficiency diseases) or prior organ transplantation;
      7) Any other malignancy within 5 years prior to the first dose of the investigational drug, excluding radically resected basal cell carcinoma of the skin and carcinoma in situ of cervix.
5. Prior treatment:
   (1) Received any other unapproved clinical trial drugs or treatments within 4 weeks prior to the first dose of the investigational drug in the study;
   (2) The time interval between the most recent anti-tumor therapy and the first dose of the investigational drug meets the following requirements for time intervals: Received anti-tumor therapies such as chemotherapy, radiotherapy, targeted therapy, immunotherapy, and other clinical trial drugs within 4 weeks prior to the first dose of the investigational drug; Received oral fluoropyrimidines, small molecule targeted drugs, or traditional Chinese medicine with anti-tumor indications within 2 weeks prior to the first dose of the investigational drug; Received palliative radiotherapy or local treatment within 2 weeks prior to the first dose of the investigational drug;
   (3) Underwent major surgery within 4 weeks prior to the first dose of the investigational drug.
6. Received potent CYP3A4 inhibitors or inducers, or P-gp inhibitors or inducers within 14 days prior to the first dose of the investigational drug;
7. Known hypersensitivity to any component of the SWY2001-Ab3-LND1002 product (antibody-conjugated toxin, antibody, toxin MMAE, etc.), or hypersensitivity to humanized monoclonal antibody products;

Comorbidities (such as poorly controlled hypertension and psychiatric disorders) or any other conditions that, in the investigator's judgment, pose a significant safety risk to the patient or interfere with study completion.

### 2.3 Dose-escalation regimen

The starting dose of SWY2001-Ab3-LND1002 was set at 0.2 mg/kg, with a predefined maximum escalated dose of 3.6 mg/kg. If dose escalation reached the predefined maximum dose group and this group demonstrated acceptable safety and tolerability, the investigator and sponsor jointly discussed and decided whether to add one or more dose groups between the maximum escalated dose group and the next lower dose group for dose exploration. For specific doses, please refer to Table 3 below:

**Table 3 Dose-escalation group design**

| Dose group | 1 | 2 | 3 | 4 | 5 | 6 |
|---|---|---|---|---|---|---|
| Escalation ratio | - | 200% | 100% | 50% | 50% | 33.3% |
| Dose (mg/kg) | 0.2 | 0.6 | 1.2 | 1.8 | 2.7 | 3.6 |

### 2.4 Experimental results

### 2.4.1 Efficacy in dose-escalation phase

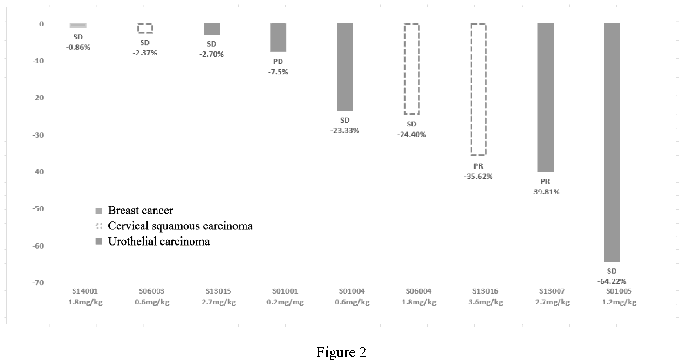

9 subjects completed at least one efficacy assessment. The assessment results for best overall response were as follows: 1 case of PD, 5 cases of SD and 3 cases of PR; PR was observed in subjects with urothelial carcinoma at 1.2 mg/kg and 2.7 mg/kg, and in a subject with cervical cancer at 3.6 mg/kg; 3.6 mg/kg was the potential target effective dose of SWY2001-Ab3-LND1002. The results are shown in Table 4 and FIG. 2.

**Table 4**

| Dose group | Subject No. | Age | Gender | Tumor type | | Nectin4 expression status | | | | First dose date | Current status | Tumor assessment results and percent change in target lesions | | | | | Best overall response assessment |
|---|---|---|---|---|---|---|---|---|---|---|---|---|---|---|---|---|---|
| | | | | | | H-score | 1+ | 2+ | 3+ | | | 1st Assessment | 2nd Assessment | 3rd Assessment | 4th Assessment | 5th Assessment | |
| 0.2 mg/kg Q3W | S01001 | 58 | Female | Urothelial carcinoma | Renal pelvic carcinoma | 80 | 30 | 10 | 10 | February 03, 2023 | Discontinued | -7.5% | 37.79% | / | | | PD |
| 0.6 mg/kg Q3W | S06003 | 61 | Female | Cervical cancer | Adenocarcinoma | 25 | 25 | 0 | 0 | March 14, 2023 | Discontinued | -2.37% | 26.48% | / | | | SD |
| | S01004 | 60 | Female | Urothelial carcinoma | Bladder cancer | 92 | 3 | 7 | 25 | March 31, 2023 | On treatment | -3.47% | -12.72% | 11.25% | -23.33% | -20.4% | SD |
| 1.2 mg/kg Q3W | S01005 | 68 | Male | Urothelial carcinoma | Bladder cancer | 175 | 40 | 15 | 35 | May 09, 2023 | Discontinued | -30% | -60% | -64.22% | / | | PR |
| 1.8 mg/kg Q3W | S06004 | 39 | Female | Cervical cancer | Squamous carcinoma | 65 | 30 | 10 | 5 | June 06, 2023 | Discontinued | -24.40% | -23.41% | 18.45% | / | | SD |
| | S14001 | 37 | Female | Breast cancer | | 65 | 30 | 10 | 5 | June 07, 2023 | Discontinued | -0.86% | 1.29% | 27.16% | / | | SD |
| 2.7 mg/kg Q3W | S13007 | 58 | Male | Urothelial carcinoma | Renal pelvic carcinoma | 190 | 20 | 40 | 30 | August 16, 2023 | On treatment | -15.04% | -38.91% | | | | PR |
| | S13015 | 64 | Male | Urothelial carcinoma | Bladder cancer | 105 | 35 | 5 | 20 | September 04, 2023 | On treatment | -1.84% | -2.70% | | | | SD |
| 3.6 mg/kg Q3W | S13016 | 52 | Female | Cervical cancer | Squamous carcinoma | 185 | 5 | 30 | 40 | October 09, 2023 | On treatment | -35.62% | | | | | PR |

### 2.4.2 Safety

No DLT events were observed in the 0.2-3.6 mg/kg dose groups. Anemia, dry eye, and corneal epithelial lesions were the most common treatment-related adverse events (TRAEs) associated with SWY2001-Ab3-LND1002. Other common TRAEs (incidence ≥20%) included proteinuria, decreased lymphocyte count, and hypoalbuminemia.

All dose groups were predominantly associated with CTCAE (Common Terminology Criteria for Adverse Events) grade 1 or 2 adverse reactions, and no CTCAE grade 4 adverse reactions occurred. One patient in the 1.8 mg/kg dose group developed a possible drug-related pulmonary infection (CTCAE grade 3) and recovered after treatment. The patient discontinued study drug administration due to possible drug-related interstitial lung disease (CTCAE grade 3). 2.4.3 PK

After a single dose of SWY2001-Ab3-LND1002 across the dose range of 0.2-3.6 mg/kg, the *in vivo* exposures of total antibody, ADC and MMAE generally increased with dose escalation. In all dose groups, the exposure of ADC was lower than that of total antibody, accounting for about 60%-80% of the total antibody exposure; the exposure of MMAE was significantly lower than that of total antibody and ADC: less than 5% of the total antibody and ADC exposure.

The elimination half-lives of total antibody, ADC, and MMAE were about 4-6 days, 4-5 days, and 5-10 days, respectively; MMAE peaked about 3-7 days after administration; no significant drug accumulation was observed after three doses administered Q3W.

At comparable doses, SWY2001-Ab3-LND1002 and MMAE exhibited slower *in vivo* elimination than PADCEV. The Cₘₐₓ of SWY2001-Ab3-LND1002 was slightly lower than that of PADCEV. Its trough concentration and AUC21d were comparable to those of PADCEV, while the MMAE exposure was significantly lower (reduced by 50% or more).

### Example 2: Updated cohorts and results of phase I clinical trial in Example 1

Example 1 provided the progress and results of the phase I clinical trial prior to the filing of the priority application for the present application. Example 2 provided newly added clinical trial data for Example 1, and a 2.4 mg/kg Q2W dose group was added. The efficacy data of some subjects are shown in Table 5. A total of 8 dose groups completed enrollment (including 2.4 mg/kg Q2W, 3.6 mg/kg Q3W, etc.).

Efficacy outcomes: As of November 27, 2024, 183 subjects were successfully screened, and 173 subjects received drug treatment. Among them, 135 subjects completed at least one tumor assessment. The overall ORR was 31.9%, including 1 case of CR, 42 cases of PR, 58 cases of SD, and 34 cases of PD; the DCR was 74.8%; the median PFS was 4.14 months; the median DoR was 6.11 months.

The efficacy of the three dose groups in the PK expansion phase was as follows: for the 2.4 mg/kg Q2W dose group, the ORR was 31.6% and the DCR was 78.9%, with the median PFS not reached; for the 2.7 mg/kg Q3W group, the ORR was 20%, the DCR was 66.7%, and the PFS was 2.76 months; for the 3.6 mg/kg Q3W group, the ORR was 34.9%, the DCR was 76.7%, and the median PFS was 4.07 months.

The predominant tumor types among enrolled subjects were urothelial carcinoma (68 cases) and cervical cancer (58 cases). Among them, 53 subjects with urothelial carcinoma and 47 subjects with cervical cancer completed at least one tumor assessment. The overall ORR for urothelial carcinoma was 37.7%, including 20 cases of PR, 19 cases of SD, and 14 cases of PD. For the 3.6 mg/kg Q3W dose group, the ORR was 40%, and the DCR was 70%. The overall ORR for cervical cancer was 36.2%, including 1 case of CR, 16 cases of PR, 22 cases of SD, and 8 cases of PD. For the 3.6 mg/kg Q3W dose group, the ORR was 38.9%, and the DCR was 83.3%.

**Table 5 Efficacy evaluation data of some patients**

| Dose group | Subject No. | Age | Gender | Tumor type | | First dose date | Current status | Best percent change in target lesions | Best overall response assessment |
|---|---|---|---|---|---|---|---|---|---|
| 3.6 mg/kg Q3W | S14012 | 60 | Female | Cervical cancer | Adenocarcinoma | October 16, 2024 | On treatment | Reduction of 34.41% | PR |
| 2.4 mg/kg Q2W | S09028 | 76 | Male | Urothelial carcinoma | Carcinoma of ureter | June 05, 2024 | On treatment | Reduction of 74.53% | PR |
| 3.6 mg/kg Q3W | S51008 | 38 | Female | Breast cancer | | November 04, 2024 | On treatment | Reduction of 44.70% | PR |
| 2.4 mg/kg Q2W | S25005 | 39 | Female | Breast cancer | | November 25, 2024 | On treatment | Reduction of 44% | PR |
| 2.4 mg/kg Q2W | S13026 | 45 | Female | Breast cancer | | May 28, 2024 | Lost to follow-up | Reduction of 23.73% | SD |
| 1.8 mg/kg Q3W | S14001 | 37 | Female | Breast cancer | | June 07, 2023 | Discontinued | Reduction of 0.86% | SD |
| 3.6 mg/kg Q3W | S13019 | 71 | Female | Breast cancer | | April 09, 2024 | Discontinued | Reduction of 8.11% | SD |
| 3.6 mg/kg Q3W | S51002 | 56 | Female | Breast cancer | | September 25, 2024 | On treatment | Reduction of 10.7% | SD |
| 3.6 mg/kg Q3W | S19003 | 74 | Female | Breast cancer | | October 19, 2024 | On treatment | Reduction of 7.8% | SD |

Safety outcomes: SWY2001-Ab3-LND1002 demonstrated favorable overall safety, as did the 3.6 mg/kg dose group. Of the 173 participants, TRAEs were predominantly grades 1-2, with only 22% experiencing grade ≥3 TRAEs and no grade 5 TRAEs. The safety of the 3.6 mg/kg Q3W dose group and other dose groups was similar to that of the overall population, likewise showing predominance of grades 1-2 events and no grade 5 TRAEs. The common TRAEs ≥20% included corneal diseases, dry eye, anemia, elevated AST, and hypertriglyceridemia. Common TRAEs in the 3.6 mg/kg Q3W dose group and other dose groups were consistent with those in the overall population.

Corneal diseases were the most common AEs and the leading cause of dose interruption. These events were predominantly grades 1-2, resolved after dose interruption and symptomatic supportive care, and did not lead to permanent discontinuation. Compared with the marketed counterpart enfortumab vedotin, SWY2001-Ab3-LND1002 showed a significant decrease in the incidence and severity of peripheral neuropathy, skin toxicity, hyperglycemia, and hematological toxicity (decreased neutrophil count, decreased white blood cell count, and decreased platelet count). Both the overall population and the 3.6 mg/kg dose group demonstrated favorable safety and tolerability.

PK outcomes: Following intravenous infusion of SWY2001-Ab3-LND1002 across the dose range of 0.2-4.5 mg/kg, the exposures of total antibody and ADC increased with dose escalation, demonstrating an overall near-linear pharmacokinetic profile. It exhibited slow *in vivo* elimination, with mean half-lives of about 4-12 days for total antibody and 4-9 days for ADC, respectively. The plasma exposure of free MMAE was low, accounting for less than 5% of the exposures of total antibody and ADC, suggesting that SWY2001-Ab3-LND1002 was relatively stable in systemic circulation. After multiple administrations of SWY2001-Ab3-LND1002 once every three or two weeks, no significant accumulation or only slight accumulation of total antibody, ADC, and free toxin was observed.

Based on the above safety, efficacy, and PK profiles, the 3.6 mg/kg Q3W dose regimen was safe and well tolerated. SWY2001-AB3-LND1002 demonstrated superior efficacy data in the treatment of urothelial carcinoma and cervical cancer, showing greater potential for development in these disease areas.

**Table 6. Sequence information of antibodies and their components involved in the present application**

| **Antibody** | **Sequence information** | |
|---|---|---|
| **Antibody CDR** | Heavy chain | |
| | CDR1-H: GYTFTSYY | SEQ ID NO.1 |
| | CDR2-H: IYPGNVNT | SEQ ID NO.2 |
| | CDR3-H: ARGIYYFDY | SEQ ID NO.3 |
| | Light chain | |
| | CDR1-L: QSVNND | SEQ ID NO.4 |
| | CDR2-L: YAS | SEQ ID NO.5 |
| | CDR3-L: HQDYSSPFT | SEQ ID NO.6 |
| A | | |
| Humanized antibody 1 SWY2001-Ab1 | SWY2001-Ab1-VH | |
| | QVQLVQSGAEVKKPGASVKVSCKASGYTFTSYYIHWVRQAPGQRLEW MGWIYPGNVNTKYNENFRDRVTITRDTSASTAYMELSSLRSEDTAVYY CARGIYYFDYWGQGTLVTVSS SEQ ID NO.7 | |
| | SWY2001-Ab1-VL | |
| | IIQMTQSPKFLSASVGDRVTITCKASQSVNNDVAWYQQKPGQSPKLLIY YASNRDTGVPDRFSGSGSGTDFTLTISSLQPEDFATYFCHQDYSSPFTFG GGTKVEIK SEQ ID NO.8 | |
| Humanized antibody 2 SWY2001-Ab2 | SWY2001-Ab2-VH | |
| | QVQLVQSGAEVKKPGASVKVSCKASGYTFTSYYIHWVRQAPGQRLEW MGWIYPGNVNTKYNENFRDRVTITRDTSASTAYMELSSLRSEDTAVYY CARGIYYFDYWGQGTLVTVSS SEQ ID NO.7 | |
| | SWY2001-Ab2-VL | |
| | AIQMTQSPSSLSASVGDRVTITCKASQSVNNDVAWYQQKPGKAPKLLI YYASNRDTGVPSRFSGSGSGTDFTLTISSLQAEDLAVYFCHQDYSSPFT FGGGTKVEIK SEQ ID NO.9 | |
| Humanized antibody 3 SWY2001-Ab3 | SWY2001-Ab3-VH | |
| | QVQLVQSGAEVKKPGASVKVSCKASGYTFTSYYIHWVRQAPGQRLEW MGWIYPGNVNTKYNENFRDRVTITRDTSASTAYMELSSLRSEDTAVYY CARGIYYFDYWGQGTLVTVSS SEQ ID NO.7 | |
| | SWY2001-Ab3-VL | |
| | AIQMTQSPSSLSASVGDRVTITCKASQSVNNDVAWYQQKPGKAPKLLI YYASNRDTGVPSRFSGSGSGTDFTLTISSLQPEDFATYFCHQDYSSPFTF GGGTKVEIK SEQ ID NO.10 | |
| Constant region | HC1 | |
| | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQ DWLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK (SEQ ID NO.11) | |
| | LC | |
| |  (SEQ ID NO.12) | |
| | HC2 | |
| | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQ DWLNGKEYKCKVSNKALPAPIEKTISKAKGQPREPQVYTLPPSREEMT KNQVSLTCLVKGFYPSDIAVEWESNGQPENNYKTTPPVLDSDGSFFLY SKLTVDKSRWQQGNVFSCSVMHEALHNHYTQKSLSLSPGK (SEQ ID NO.13) | |

## Claims

1. Use of an antibody-drug conjugate targeting Nectin-4 or a pharmaceutically acceptable salt thereof in the manufacture of a medicament for the treatment of a tumor, wherein the tumor is preferably a Nectin-4-positive tumor, and the antibody-drug conjugate is prepared by conjugating a drug-linker represented by formula I with an antibody targeting Nectin-4 or an antigen-binding fragment thereof:

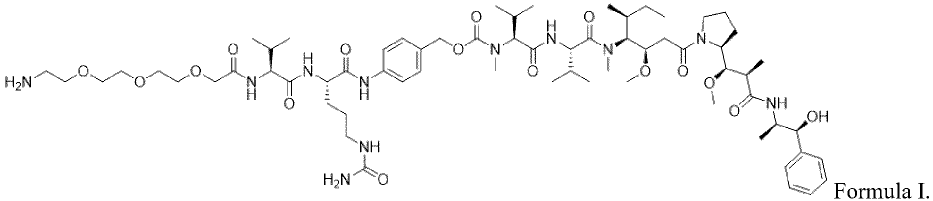

2. The use according to claim 1, wherein the antibody or the antigen-binding fragment thereof comprises a heavy chain and a light chain, wherein:
(i) the heavy chain comprises three CDR regions which are a CDR1-H region, a CDR2-H region and a CDR3-H region, and the CDR1-H region, CDR2-H region and CDR3-H region have the amino acid sequences as set forth in SEQ ID NOs: 1, 2 and 3, respectively; and/or
(ii) the light chain comprises three CDR regions which are a CDR1-L region, a CDR2-L region and a CDR3-L region, and the CDR1-L region, CDR2-L region and CDR3-L region have the amino acid sequences as set forth in SEQ ID NOs: 4, 5 and 6, respectively.

3. The use according to claim 1 or 2, wherein the antibody or the antigen-binding fragment thereof comprises a heavy chain variable region and a light chain variable region, wherein the heavy chain variable region has the amino acid sequence as set forth in SEQ ID NO: 7, and the light chain variable region has the amino acid sequence as set forth in any one of SEQ ID NOs: 8-10.

4. The use according to any one of claims 1-3, wherein the antibody or the antigen-binding fragment thereof comprises a heavy chain constant region and a light chain constant region, wherein the heavy chain constant region has the amino acid sequence as set forth in SEQ ID NO: 11 or SEQ ID NO: 13, and the light chain constant region has the amino acid sequence as set forth in SEQ ID NO: 12.

5. The use according to any one of claims 1-4, wherein the tumor is selected from the group consisting of cervical cancer (such as cervical adenocarcinoma or cervical squamous carcinoma), urothelial carcinoma (such as bladder cancer, renal pelvic carcinoma, carcinoma of urethra, or carcinoma of ureter), and breast cancer.

6. The use according to any one of claims 1-5, wherein the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg.

7. The use according to any one of claims 1-6, wherein the antibody-drug conjugate or the pharmaceutically acceptable salt thereof is administered at a frequency of once weekly (QW), once every two weeks (Q2W), once every three weeks (Q3W), or once every four weeks (Q4W).

8. The use according to any one of claims 1-7, wherein based on the weight of the antibody-drug conjugate, a unit preparation of the medicament contains the antibody-drug conjugate or the pharmaceutically acceptable salt thereof in an amount of about 1 mg to about 500 mg, preferably about 5 mg to about 400 mg, or about 10 mg to about 300 mg, or about 10 mg to about 250 mg, or about 10 mg to about 200 mg, or about 20 mg to about 200 mg, more preferably about 10 mg to about 160 mg, or about 20mg to about 160 mg, or about 80 mg to about 160 mg; or about 120 mg to about 240 mg, or about 160 mg to about 220 mg; for example, about 10 mg, about 20 mg, about 30 mg, about 40 mg, about 60 mg, about 80 mg, about 100 mg, about 120 mg, about 140 mg, or about 160 mg, more preferably about 10 mg, about 20 mg, about 40 mg, about 80 mg, about 120 mg, or about 160 mg, further more preferably about 20 mg, about 40 mg, about 80 mg, about 120 mg, about 160 mg, or about 240 mg, and further more preferably about 10 mg or about 40 mg.

9. The use according to claim 8, wherein based on the weight of the antibody-drug conjugate, the unit preparation contains the antibody-drug conjugate or the pharmaceutically acceptable salt thereof in an amount of 120 mg to 300 mg, preferably about 120 mg, about 130 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, or about 300 mg.

10. The use according to any one of claims 1-9, wherein the medicament is prepared into a clinically acceptable preparation, such as an oral preparation, an injectable preparation, a topical preparation, or an external preparation.

11. A method for treating a tumor, comprising administering to a subject or patient in need thereof a therapeutically effective amount of an antibody-drug conjugate targeting Nectin-4 or a pharmaceutically acceptable salt thereof, wherein the tumor is preferably a Nectin-4-positive tumor, and the antibody-drug conjugate is prepared by conjugating a drug-linker represented by formula I with an antibody targeting Nectin-4 or an antigen-binding fragment thereof:

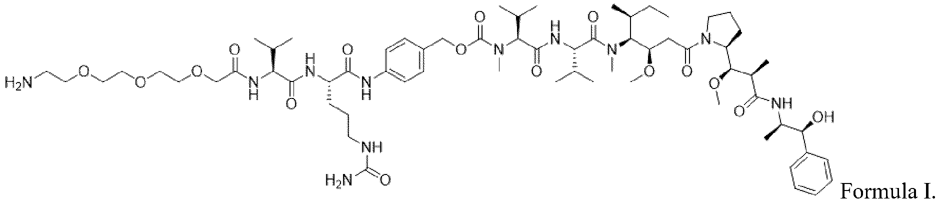

12. The method according to claim 11, wherein the antibody or the antigen-binding fragment thereof comprises a heavy chain and a light chain, wherein:
(i) the heavy chain comprises three CDR regions which are a CDR1-H region, a CDR2-H region and a CDR3-H region, and the CDR1-H region, CDR2-H region and CDR3-H region have the amino acid sequences as set forth in SEQ ID NOs: 1, 2 and 3, respectively; and/or
(ii) the light chain comprises three CDR regions which are a CDR1-L region, a CDR2-L region and a CDR3-L region, and the CDR1-L region, CDR2-L region and CDR3-L region have the amino acid sequences as set forth in SEQ ID NOs: 4, 5 and 6, respectively.

13. The method according to claim 11 or 12, wherein the antibody or the antigen-binding fragment thereof comprises a heavy chain variable region and a light chain variable region, wherein the heavy chain variable region has the amino acid sequence as set forth in SEQ ID NO: 7, and the light chain variable region has the amino acid sequence as set forth in any one of SEQ ID NOs: 8-10.

14. The method according to any one of claims 11-13, wherein the antibody or the antigen-binding fragment thereof comprises a heavy chain constant region and a light chain constant region, wherein the heavy chain constant region has the amino acid sequence as set forth in SEQ ID NO: 11 or SEQ ID NO: 13, and the light chain constant region has the amino acid sequence as set forth in SEQ ID NO: 12.

15. The method according to any one of claims 11-14, wherein the tumor is selected from the group consisting of cervical cancer (such as cervical adenocarcinoma or cervical squamous carcinoma), urothelial carcinoma (such as bladder cancer, renal pelvic carcinoma, carcinoma of urethra, or carcinoma of ureter), and breast cancer.

16. The method according to any one of claims 11-15, wherein the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg.

17. The method according to any one of claims 11-16, wherein the antibody-drug conjugate or the pharmaceutically acceptable salt thereof is administered at a frequency of once weekly (QW), once every two weeks (Q2W), once every three weeks (Q3W), or once every four weeks (Q4W).

18. The method according to any one of claims 11-17, wherein based on the weight of the antibody-drug conjugate, the therapeutically effective amount of the antibody-drug conjugate or the pharmaceutically acceptable salt thereof is about 1 mg to about 500 mg, preferably about 5 mg to about 400 mg, or about 10 mg to about 300 mg, or about 10 mg to about 250 mg, or about 10 mg to about 200 mg, or about 20 mg to about 200 mg, more preferably about 10 mg to about 160 mg, or about 20mg to about 160 mg, or about 80 mg to about 160 mg; or about 120 mg to about 240 mg, or about 160 mg to about 220 mg; for example, about 10 mg, about 20 mg, about 40 mg, about 60 mg, about 80 mg, about 100 mg, about 120 mg, about 140 mg, or about 160 mg, more preferably about 10 mg, about 20 mg, about 40 mg, about 80 mg, about 120 mg, or about 160 mg, further more preferably about 20 mg, about 40 mg, about 80 mg, about 120 mg, about 160 mg, or about 240 mg, and further more preferably about 10 mg or about 40 mg.

19. The method according to claim 18, wherein the therapeutically effective amount is about 90 mg to 350 mg, for example: about 90 mg, about 100 mg, about 110 mg, about 120 mg, about 130 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, about 300 mg, about 305 mg, about 310 mg, about 320 mg, about 333 mg, about 340 mg, or about 350 mg.

20. The method according to any one of claims 11-19, wherein the antibody-drug conjugate or the pharmaceutically acceptable salt thereof is prepared into a clinically acceptable preparation, such as an oral preparation, an injectable preparation, a topical preparation, or an external preparation.

21. A pharmaceutical composition comprising (i) an antibody-drug conjugate targeting Nectin-4 or a pharmaceutically acceptable salt thereof and (ii) a pharmaceutically acceptable carrier, for use in the treatment of a tumor (including a cancer), wherein the tumor is preferably a Nectin-4-positive tumor, and the antibody-drug conjugate is prepared by conjugating a drug-linker represented by formula I with an antibody targeting Nectin-4 or an antigen-binding fragment thereof:

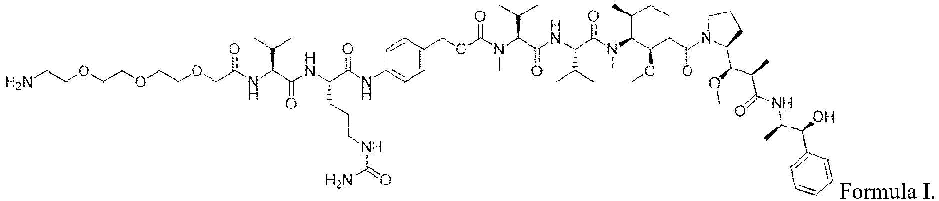

22. The pharmaceutical composition for use according to claim 21, wherein the antibody or the antigen-binding fragment thereof comprises a heavy chain and a light chain, wherein:
(i) the heavy chain comprises three CDR regions which are a CDR1-H region, a CDR2-H region and a CDR3-H region, and the CDR1-H region, CDR2-H region and CDR3-H region have the amino acid sequences as set forth in SEQ ID NOs: 1, 2 and 3, respectively; and/or
(ii) the light chain comprises three CDR regions which are a CDR1-L region, a CDR2-L region and a CDR3-L region, and the CDR1-L region, CDR2-L region and CDR3-L region have the amino acid sequences as set forth in SEQ ID NOs: 4, 5 and 6, respectively.

23. The pharmaceutical composition for use according to claim 21 or 22, wherein the antibody or the antigen-binding fragment thereof comprises a heavy chain variable region and a light chain variable region, wherein the heavy chain variable region has the amino acid sequence as set forth in SEQ ID NO: 7, and the light chain variable region has the amino acid sequence as set forth in any one of SEQ ID NOs: 8-10.

24. The pharmaceutical composition for use according to any one of claims 21-23, wherein the antibody or the antigen-binding fragment thereof comprises a heavy chain constant region and a light chain constant region, wherein the heavy chain constant region has the amino acid sequence as set forth in SEQ ID NO: 11 or SEQ ID NO: 13, and the light chain constant region has the amino acid sequence as set forth in SEQ ID NO: 12.

25. The pharmaceutical composition for use according to any one of claims 21-24, wherein the tumor is selected from the group consisting of cervical cancer (such as cervical adenocarcinoma or cervical squamous carcinoma), urothelial carcinoma (such as bladder cancer, renal pelvic carcinoma, carcinoma of urethra, or carcinoma of ureter), and breast cancer.

26. The pharmaceutical composition for use according to any one of claims 21-25, wherein the antibody-drug conjugate or the pharmaceutically acceptable salt thereof is administered at a dosage ranging from about 0.03 mg/kg to about 20.0 mg/kg per administration, based on the weight of the antibody-drug conjugate, preferably from about 1.0 mg/kg to about 10.0 mg/kg, and more preferably from about 2.0 mg/kg to about 8.0 mg/kg, for example: about 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.5 mg/kg, 5.0 mg/kg, 5.5 mg/kg, or 6.0 mg/kg.

27. The pharmaceutical composition for use according to any one of claims 21-26, wherein the pharmaceutical composition is administered at a frequency of once weekly (QW), once every two weeks (Q2W), once every three weeks (Q3W), or once every four weeks (Q4W).

28. The pharmaceutical composition for use according to any one of claims 21-27, wherein based on the weight of the antibody-drug conjugate, a unit preparation of the pharmaceutical composition contains the antibody-drug conjugate or the pharmaceutically acceptable salt thereof in an amount of about 1 mg to about 500 mg, preferably about 5 mg to about 400 mg, or about 10 mg to about 300 mg, or about 10 mg to about 250 mg, or about 10 mg to about 200 mg, or about 20 mg to about 200 mg, more preferably about 10 mg to about 160 mg, or about 20mg to about 160 mg, or about 80 mg to about 160 mg; or about 120 mg to about 240 mg, or about 160 mg to about 220 mg; for example, about 10 mg, about 20 mg, about 30 mg, about 40 mg, about 60 mg, about 80 mg, about 100 mg, about 120 mg, about 140 mg, or about 160 mg, more preferably about 10 mg, about 20 mg, about 40 mg, about 80 mg, about 120 mg, or about 160 mg, further more preferably about 20 mg, about 40 mg, about 80 mg, about 120 mg, about 160 mg, or about 240 mg, and further more preferably about 10 mg or about 40 mg.

29. The pharmaceutical composition for use according to claim 28, wherein based on the weight of the antibody-drug conjugate, the unit preparation contains the antibody-drug conjugate or the pharmaceutically acceptable salt thereof in an amount of 120 mg to 300 mg, preferably about 120 mg, about 130 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, or about 300 mg.

30. The pharmaceutical composition for use according to any one of claims 21-29, wherein the pharmaceutical composition is prepared into a clinically acceptable preparation, such as an oral preparation, an injectable preparation, a topical preparation, or an external preparation.
